# EUROPEAN PATENT SPECIFICATION

(11) **EP 0 769 962 B1**
(45) Date of publication and mention of the grant of the patent: **26.03.2003**
(21) Application number: 95925386.5
(22) Date of filing: 06.07.1995
(51) Int. Cl.: A61K 38/57, A61P 17/00

(54) **METHOD OF ATTENUATING ARTERIAL STENOSIS**
METHODE ZUR VERMINDERUNG ARTERIELLER STENOSE
PROCEDE PERMETTANT D'ATTENUER LES STENOSES ARTERIELLES

(30) Priority: 07.07.1994 US 271930
(43) Date of publication of application: 02.05.1997
(73) Proprietor: WASHINGTON UNIVERSITY, St. Louis, MO 63110 (US)
(72) Inventor: ABENDSCHEIN, D. Washington Uty. School of Medicine, St. Louis, MO 63110 (US)
(74) Representative: Strych, Werner Dr.
(86) International application number: US9508221
(87) International publication number: WO96001649

(56) References cited:
- EP-A- 0 473 564
- EP-A- 0 563 023
- WO-A-93/25230
- CIRCULATION, vol. 84, no. 2, 1991 NEW YORK, pages 821-827, HASKEL E.J. ET AL 'Prevention of arterial reocclusion after thrombolysis with recombinant lipoprotein-associated coagulation inhibitor'
- CIRCULATION, vol. 90, no. 4, October 1994 NEW YORK, page I344 OLTRONA L. ET AL 'Inhibition of Tissue factor mediated thrombosis markedly attenuates stenosis after ballon-induced arterial injury in hyperlipidemic minipigs'

## Description

### Background of the Invention

This invention claims the use according to claim 1. It relates to a novel method of attenuating arterial stenosis. More particularly, the invention relates to the use according to claim 1 in a method of reducing or inhibiting restenosis following balloon angioplasty by the administration of a blood coagulation inhibitor known as lipoprotein-associated coagulation inhibitor (LACI) and alternatively as tissue factor pathway inhibitor (TFPI).

This invention was made with government support under grant number HL 17464 awarded by the National Institutes of Health. The government has certain rights in the invention.

Balloon angioplasty is a widely used medical procedure for treatment of arterial blockage. However, early thrombotic re-occlusion of treated arteries occurs in approximately 5% of patients as reported by Ip et al., J.Am.Col.Cardiol. 17, 77B-88B (1991). Furthermore, stenosis, which is a narrowing or stricture of the artery, reoccurs within three (3) months in up to 50% of arteries successfully recanalized by angioplasty as reviewed by Ip et al., J.Am.Col.Cardiol. 17, 77B-88B (1991), and by Franklin et al., Coronary Artery Dis. 4, 232-42 (1993). The reocclusion and restenosis following balloon angioplasty thus necessitates repeat angioplasty in many patients as described by Holmes et al., Am.J.Cardiol. 53, 77C (1984), and Lincoff et al., J.Am.Col.Cardiol. 19, 926-35 (1992). Accordingly, a method of attenuating stenosis after balloon angioplasty would have significant utility in medical practice.

Anticoagulation treatment with intravenous heparin is standard clinical practice to inhibit thrombosis during and after angioplasty. Despite its effect to inhibit thrombin, and evidence that it may inhibit smooth muscle cell proliferation, however, heparin does not appear to attenuate restenosis in patients as reported by Ellis et al., Am. Heart J. 117, 777-82 (1989). Similarly, in experimental animals, Gimple et al. reported (Circulation 86, 1536-46, 1992) that chronic, subcutaneous administration of heparin did not improve the absolute luminal diameter after balloon angioplasty.

Other anticoagulants have been tested to inhibit restenosis, but the results are inconclusive. Administration of the oral anticoagulant warfarin did not decrease rates of restenosis in patients as described by Thornton et al., Circulation 69, 721-7 (1984), and Urban et al., Br. Heart J. 60, 485-8 (1988). Direct inhibition of thrombin with recombinant desulfatohirudin administered for only two (2) hours after angioplasty of iliac arteries in rabbits decreased luminal stenosis one month later according to Sarembock et al., Circulation 84, 232-43 (1991). However, Webster et al. (Circulation 84, II580, 1991) showed that the same inhibitor infused continuously for two (2) weeks after balloon-induced arterial injury in pigs did not attenuate subsequent luminal stenosis. Nevertheless, inhibition of factor X, with low molecular weight heparin was shown to decrease intimal proliferation following balloon angioplasty in rabbits as reported by Hanke et al., Circulation 85, 1548-56 (1992).

Other proposed drug treatments include agents that block the platelet receptor known as glycoprotein IIb/IIIa. One such agent designated CentoRx (also known as 7E3) is described in The New England Journal of Medicine, April 7, 1994, and The Lancet, April 9, 1994. Other antithrombotics, e.g. argatroban (Novastan), described in U.S. Patent 4,248,192, and Hirulog, an analog of hirudin, also have been proposed for preventing restenosis. U.S. Patent 5,308,622 describes the use of a conjugate of basic fibroblast growth factor (bFGF) and saporin (a ribosome inactivating agent) for preventing restenosis.

It is known that plasma contains a multivalent Kunitz-type inhibitor of coagulation, referred to herein as tissue factor pathway inhibitor (TFPI). This name has been accepted by the International Society on Thrombosis and Kemostasis, June 30, 1991, Amsterdam. TFPI was first purified from a human hepatoma cell, Hep G2, as described by Broze and Miletich, Proc. Natl. Acad. Sci. USA 84, 1886-1890 (1987), and subsequently from human plasma as reported by Novotny et al., J.Biol.Chem. 264, 18832-18837 (1989); and Chang liver and SK hepatoma cells as disclosed by Wun, et al., J.Biol.Chem. 265, 16096-16101 (1990). TFPI cDNA have been isolated from placental and endothelial cDNA libraries as described by Wun et al., J.Biol.Chem. 263, 6001-6004 (1988); and Girard et al., Thromb. Res. 55, 37-50 (1989). The primary amino acid sequence of TFPI, deduced from the cDNA sequence, shows that TFPI contains a highly negatively charged amino-terminus, three tandem Kunitz-type inhibitory domains, and a highly positively charged carboxyl terminus. The first Kunitz-domain of TFPI is needed for the inhibition of the factor VIIₐ/tissue factor complex, and the second Kunitz-domain of TFPI is responsible for the inhibition of factor X, according to Girard et al., Nature 328, 518-520 (1989), while the function of the third Kunitz-domain remains unknown. See also U.S. Patent 5,106,833. TFPI is believed to function in vivo to limit the initiation of coagulation by forming an inert, quaternary factor Xₐ: TFPI: factor VIIₐ: tissue factor complex. Further background information on TFPI can be had by reference to the recent reviews by Rapaport, Blood 73, 359-365 (1989); and Broze et al., Biochemistry 29, 7539-7546 (1990).

Recombinant TFPI has been expressed as a glycosylated protein using mammalian cell hosts including mouse C127 cells as disclosed by Day, et al., Blood 76, 1538-1545 (1990), baby hamster kidney cells as reported by Pedersen, et al., J.Biol.Chem. 265, 16786-16793 (1990), Chinese hamster ovary cells and human SK hepatoma cells. The C127 TFPI has been used in animal studies and shown to be effective in the inhibition of tissue factor-induced intravascular coagulation in rabbits according to Day, et al., supra, and in the prevention of arterial reocclusion after thrombolysis in dogs as described by Haskel, et al., Circulation 84, 821-827 (1991).

Recombinant TFPI also has been expressed as a non-glycosylated protein using E. coli host cells and obtaining a highly active TFPI by in vitro folding of the protein as described in U.S. Patent 5,212,091.

See also Wun, et al., Thromb. Hemostas. 68, 54-59 (1992).

The cloning of the TFPI cDNA which encodes the 276 amino acid residue protein of TFPI is further described in Wun, et al., U.S. Patent 4,966,852.

Recently, TFPI obtained through recombinant DNA clones expressed in E. coli as disclosed in U.S. Patent 5,212,091 has been described as useful for reducing the thrombogenicity of microvascular anastomoses. See U.S. Patent 5,276,015.

### Brief Description of the Invention

In accordance with the present invention the use according to claim 1 is claimed. A novel method is provided for attenuating stenosis after balloon angioplasty. The method is for parenterally administering to a warm-blooded mammal following balloon angioplasty an effective amount of tissue factor pathway inhibitor (TFPI) sufficient to reduce the extent of stenosis.

Because TFPI inhibits both factor Xₐ elaborated by the complex of tissue factor and factor VIIₐ as well as the activity of the complex, which is induced after injury to the vessel, TFPI is believed to have advantages over single site inhibitors including those for thrombin (the product of activation of prothrombin by factor Xₐ) or factor Xₐ. The results described herein show the advantage of TFPI compared with heparin for inhibition of stenosis after vessel injury.

The invention is illustrated in particular hereinbelow by the intravenous administration of the TFPI after balloon hyperinflation-induced injury in the carotid artery of minipigs with hypercholesteremia induced by atherogenic diet.

It will be appreciated that although the method of the invention is illustrated in particular hereinbelow with the minipig species, it is also useful for other warm-blooded mammals, e.g., humans, in an analogous manner.

As defined herein, TFPI can be either glycosylated or non-glycosylated.

### Detailed Description of the Invention

While the specification concludes with claims particularly pointing out and distinctly claiming the subject matter which is regarded as forming the invention, it is believed that the invention will be better understood from the following detailed description of preferred embodiments of the invention taken in conjunction with the accompanying drawings in which:

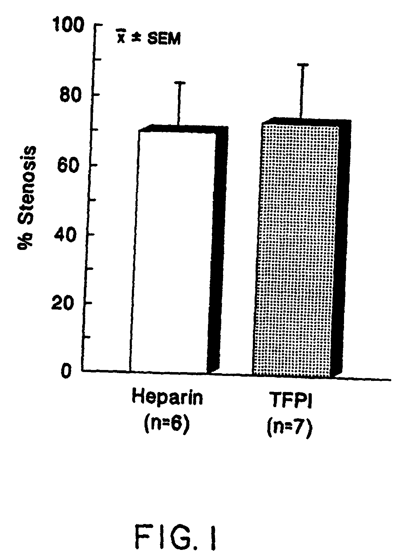

FIG. 1 is a bar graph which shows stenosis (in percentage) of the carotid arterial lumen four (4) weeks after balloon hyperinflation-induced injury in minipigs given intravenous infusions for three (3) hours of either heparin (100 U/kg/h) or TFPI (0.5 mg/kg bolus and 6 mg/kg/h) beginning at the time of vessel injury.

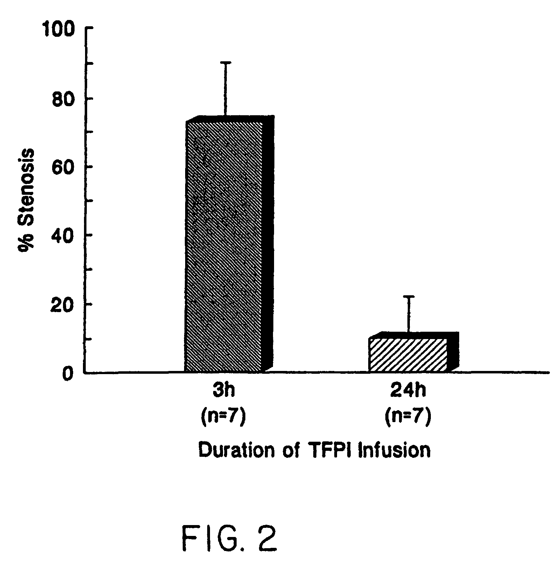

FIG. 2 is a bar graph which compares stenosis (in percentage) of the carotid artery four (4) weeks after balloon-induced injury in minipigs given TFPI as an intravenous infusion (0.5 mg/kg bolus and 6 mg/kg/h) for either three (3) hours or 24 hours.

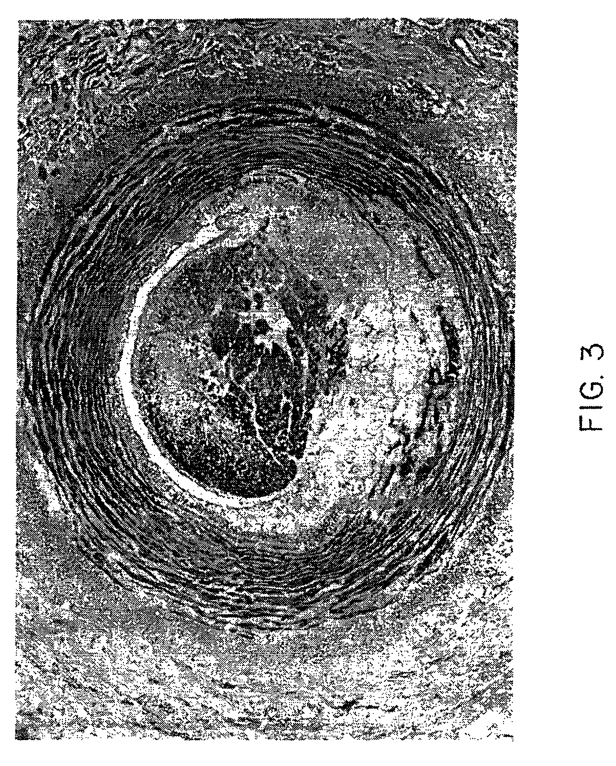

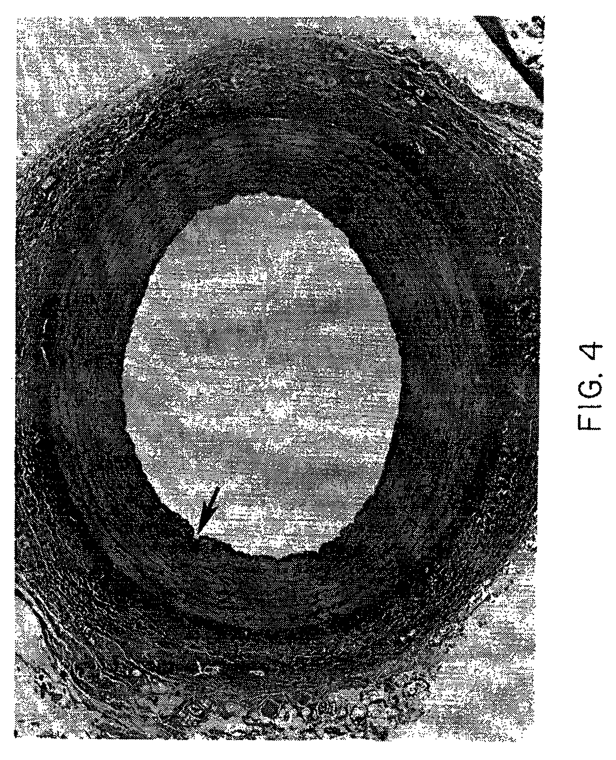

FIG. 3 is a photomicrograph of a carotid arterial cross-section stained with Masson's trichrome for collagen obtained four (4) weeks after balloon-induced injury in a minipig given heparin. Stenosis of the lumen is >80% with the intimal lesion comprised of a fibrous cap containing smooth muscle cells, a core of organizing thrombus, and foam cells at the base of the lesion. Severe damage to the media is evident by replacement of smooth muscle cells with collagen. The magnification in FIG. 3 and FIG. 4 is 60 power.
FIG. 4 is a photomicrograph of a carotid cross-section stained with Verhoeff's Van Gieson stain for elastic tissue obtained four (4) weeks after balloon injury in a minipig given TFPI for 24 hours. Despite the break in the internal elastic lamina (arrow), indicative of severe vessel injury, there is minimal proliferation of the intima and no visible thrombus on the luminal surface.

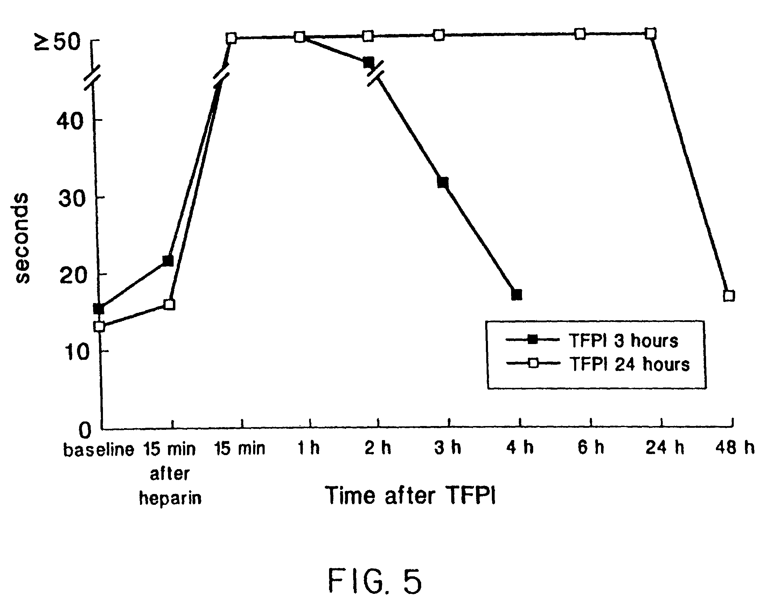

FIG. 5 is a graphical representation which shows the prothrombin time (PT)in minipigs given TFPI. The PT was ≥50 seconds (the maximum reading on the coagulation timer) throughout the infusion.

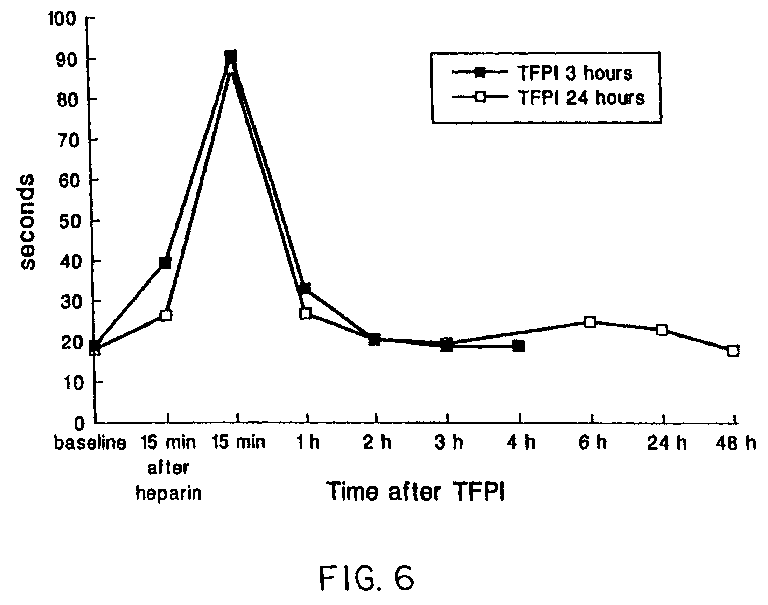

FIG. 6 is a graphical representation which shows the activated partial thromboplastin time (aPTT) in minipigs given TFPI. The early increase reflects intravenous injection of heparin given to prevent clot formation in the catheters before administration of TFPI. After heparin cleared from the circulation, but during continued infusion of TFPI, aPTT returned to baseline.

In order to illustrate the invention in greater detail, the following illustrative Example using a hyperlipidemic minipig model was carried out. It will be appreciated, however, that the invention is not limited to this exemplary work or to the specific details set forth in this Example.

### EXAMPLE

### Materials and Methods

Recombinant TFPI in 300 mM arginine phosphate buffer at pH 7.2 was infused intravenously (0.5 mg/kg bolus followed by 6 mg/kg/hr) for three (3) hrs (n=7) or for 24 hrs (n=7) after balloon hyperinflation-induced injury in the carotid arteries of minipigs with hypercholesteremia induced by atherogenic diet, which is a procedure that rapidly yields complex plaque-like lesions as reported previously in rabbits by Stevens et al., Ann. of Vascular Surgery 6, 55-61 (1992). After four (4) weeks, the injured vessels were perfusion fixed in situ and examined histologically.

The TFPI used in the foregoing Example was obtained through recombinant DNA clones expressed in E. coli. It is a 277-amino acid protein consisting of the 276 residue sequence described by Wun, et al., J.Biol.Chem. 263, 6001-6004 (1988), and in U.S. Patent 4,966,852, with an additional alanine residue inserted at the N-terminus as described in U.S. Patent 5,212,091. Unfractionated heparin was purchased from Elkins-Sinn, Cherry Hill, NJ.

### Results

Histological examination of the above-treated vessels revealed that luminal stenosis was 73±13 (SE)% in minipigs given TFPI and 70 ± 14% for those given heparin for three (3) hours (FIG. 1, p=NS). Stenosis was only 13 ± 12% in minipigs given TFPI for 24 hours (FIG. 2, p=0.008 compared with three (3) hours of TFPI). Intimal lesions in animals receiving TFPI for 24 hours were negligible despite severe vessel injury indicated by rupture of the internal elastic lamina (FIG. 4) Adherent thrombus and foam cells observed in heparin-treated animals (FIG. 3) were also negligible in those given TFPI for 24 hours. Prothrombin time (PT), which evaluates the extrinsic pathway of coagulation by addition of tissue factor and calcium to a sample of plasma with measurement of the time of clot formation as the endpoint, was >2.5-fold baseline throughout the infusion of TFPI (FIG. 5). However, no changes in activated partial thromboplastin time (aPTT), which measures the intrinsic pathway of coagulation, were observed (FIG. 6). Thus, a 24-hr, but not a 3-hr infusion of TFPI markedly attenuated stenosis after balloon-induced arterial injury. These data indicate that TF-mediated thrombosis plays a role in the stenosis of injured vessels and that its prolonged inhibition can limit restenosis after balloon angioplasty.

The parenteral administration of the TFPI is preferably carried out by intravenous administration of the TFPI from admixture with a physiologically acceptable vehicle or carrier, e.g., normal saline or buffered saline such as phosphate buffered saline (PBS), arginine phosphate buffer or other such pharmaceutically acceptable buffers, e.g., HEPES. Such conventional vehicles are well known, as can be seen by reference to numerous texts and treatises in the field of drug administration, e.g., Remington's Pharmaceutical Sciences, ed. Arthur Osol, 16th ed., 1980, Mack Publishing Co., Easton, PA. The amount of TFPI parenterally administered can vary widely, depending upon the degree or severity of the stenosis. Doses of from about 0.5 mg/kg to about 6.0 mg/kg per hr by prolonged administration of from three (3) to about 24 hrs are preferred. Intermittent bolus injections of TFPI in the range of 0.5 mg/kg to 1.0 mg/kg, (or direct delivery of TFPI to the vessel with use of catheter-based drug delivery systems at critical intervals after induction of vascular injury) e.g., at zero (0) hrs and eight (8) hrs may reduce the total quantity of drug required.

## Claims

1. Use of an effective amount of Tissue Factor Pathway Inhibitor (TFPI) sufficient to reduce the extent of stenosis following balloon angioplasty for preparing a medicament for parenteral administration for attenuating stenosis after balloon angioplasty in a warm blooded mammal.

2. Use according to Claim 1 in which the TFPI is carried in a normal saline vehicle buffered to physiological pH.

3. Use according to Claim 1 in which the TFPI is for administration in an amount of from 0.5 mg/kg to 6 mg/kg by prolonged administration of from 3 firs to 24 hrs.

4. Use of an effective amount of TFPI sufficient to reduce the extent of stenosis in a warm blooded mammal following balloon angioplasty, for preparing a medicament for administration over a prolonged period of time of an effective amount of TFPI to reduce said stenosis.

5. Use according to Claim 4 wherein said TFPI is to be administered parenterally.

6. Use according to Claim 4 wherein said TFPI is to be administered by direct delivery of TFPI to the vessel.

7. Use according to Claim 4 wherein said TFPI is carried by a physiologically acceptable vehicle.

8. Use according to Claim 4 wherein said TFPI is to be administered in an amount of from 0.5 mg/kg to 6 mg/kg per hour in said physiologically acceptable vehicle.

9. Use according to Claim 4 wherein said TFPI is recombinant (ala-1)-TFPI (1-277) or TFPI (1-276).

10. Use according to Claim 4 further comprising an additional alanine at the N-terminus of said TFPI, wherein said TFPI is recombinant.

11. Use according to Claim 4 wherein said prolonged period is from about 3 hours to 24 hours.

## Patentansprüche

1. Verwendung einer wirksamen Menge eines Stoffwechselgewebsfaktorinhibitors (TFPI), die zur Verringerung der Ausbreitung von auf die Ballonangioplastie folgenden Stenosen ausreichend ist, zur Herstellung eines Medikaments zur parenteralen Verabreichung zur Verminderung der nach der Ballonangioplastie in einem warmblütigen Säugetier auftretenden Stenosen.

2. Verwendung nach Anspruch 1, wobei der TFPI von einer normalen auf einen physiologischen pH gepufferten Kochsalzträgerlösung getragen wird.

3. Verwendung nach Anspruch 1, wobei der TFPI in einer Menge von 0,5 mg/kg bis 6 mg/kg durch verlängerte Verabreichung während 3 Stunden bis 24 Stunden verabreicht wird.

4. Verwendung einer wirksamen Menge eines TFPI, die zur Verringerung der Ausbreitung von auf die Ballonangioplastie folgenden Stenosen bei warmblütigen Säugetieren ausreichend ist, zur Herstellung eines Medikaments zur Verabreichung einer wirksamen Menge an TFPI über einen ausgedehnten Zeitraum zur Verringerung solcher Stenosen.

5. Verwendung nach Anspruch 4, worin der TFPI parenteral verabreicht wird.

6. Verwendung nach Anspruch 4, worin der TFPI durch direkte Freigabe des TFPI zu dem Behälter verabreicht wird.

7. Verwendung nach Anspruch 3, worin der TFPI von einem physiologisch annehmbaren Träger getragen wird.

8. Verwendung nach Anspruch 4, worin der TFPI in einer Menge von 0,5 mg/kg bis 6 mg/kg pro Stunde in dem physiologisch annehmbaren Träger verabreicht wird.

9. Verwendung nach Anspruch 4, worin der TFPI rekombinantes (ala-1)-TFPI(1-277) oder TFPI(1-276) ist.

10. Verwendung nach Anspruch 4, weiter umfassend ein zusätzliches Alanin am N-Terminus des TFPI, worin der TFPI rekombinant ist.

11. Verwendung nach Anspruch 4, worin der verlängerte Zeitraum etwa 3 Stunden bis 24 Stunden beträgt.

## Revendications

1. Utilisation d'une quantité efficace d'Inhibiteur de la Voie du Facteur Tissulaire (TFPI) suffisante pour réduire l'étendue d'une sténose suite à une angioplastie transluminale percutanée, pour la préparation d'un médicament destiné à une administration parentérale pour atténuer une sténose après une angioplastie transluminale percutanée chez un mammifère à sang chaud.

2. Utilisation selon la revendication 1, dans laquelle le TFPI est transporté dans un véhicule salin normal tamponné au pH physiologique.

3. Utilisation selon la revendication 1, dans laquelle le TFPI est destiné à être administré dans une quantité comprise entre 0,5 mg/kg et 6 mg/kg par une administration prolongée d'une durée comprise entre 3 heures et 24 heures.

4. Utilisation d'une quantité efficace de TFPI suffisante pour réduire l'étendue d'une sténose chez un mammifère à sang chaud suite à une angioplastie transluminale percutanée, pour la préparation d'un médicament destiné à une administration sur une période de temps prolongée d'une quantité efficace de TFPI pour réduire ladite sténose.

5. Utilisation selon la revendication 4, dans laquelle ledit TFPI doit être administré par voie parentérale.

6. Utilisation selon la revendication 4, dans laquelle ledit TFPI doit être administré par une distribution directe du TFPI au vaisseau.

7. Utilisation selon la revendication 4, dans laquelle ledit TFPI est transporté par un véhicule physiologiquement acceptable.

8. Utilisation selon la revendication 4, dans laquelle ledit TFPI doit être administré dans une quantité comprise entre 0,5 mg/kg et 6 mg/kg par heure dans ledit véhicule physiologiquement acceptable.

9. Utilisation selon la revendication 4, dans laquelle ledit TFPI est (ala-1)-TFPI (1-277) recombinant ou TFPI (1-276).

10. Utilisation selon la revendication 4, comprenant en outre une alanine supplémentaire à l'extrémité N terminale dudit TFPI, dans laquelle ledit TFPI est recombinant.

11. Utilisation selon la revendication 4, dans laquelle ladite période prolongée est comprise entre 3 heures et 24 heures.
